# EUROPEAN PATENT APPLICATION

(11) **EP 4 219 737 A1**
(43) Date of publication of application: **02.08.2023**
(21) Application number: 20954447.7
(22) Date of filing: 23.09.2020
(51) Int. Cl.: C12Q 1/68

(54) **COMBINATION, METHOD AND KIT FOR DETECTING NUCLEIC ACID**

(71) Applicant: Maccura Biotechnology Co., Ltd., Chengdu Sichuan 611731 (CN)
(72) Inventor: ZHAO, Yuhang, Chengdu, Sichuan 611731 (CN); HUANG, Qiuping, Chengdu, Sichuan 611731 (CN); HE, Huihuang, Chengdu, Sichuan 611731 (CN)
(74) Representative: Dr. Gassner & Partner mbB
(86) International application number: PCT/CN2020/117163
(87) International publication number: WO 2022/061593

(57) **Abstract**

A combination, a method and a kit for detecting nucleic acids. The combination comprises: an upstream primer, wherein a target sequence binding region is provided at the 3'-end thereof, and the 3'-end of the target sequence binding region is capable of being complementary to a specific detection site of a target sequence; a downstream primer, wherein a target sequence binding region is provided at the 3'-end thereof, and the 3'-end of the target sequence binding region is capable of being complementary to the same specific detection site; and a signal oligonucleotide, wherein the signal oligonucleotide is modified with a first detection group and a second detection group, and the first detection group and the second detection group generate a change in signal by means of a change in distance.

## Description

### TECHNICAL FIELD

The present invention relates to the field of molecular biology, and in particular to the detection of nucleic acids.

### BACKGROUND

Histopathologic diagnosis is a gold standard for tumor diagnosis and a basis for clinical treatment for a long time. However, pathological tissues are difficult to obtain, the sampling thereof is thus inconvenient, and it is hard to continuously perform sampling to constantly monitor a genetic mutation condition of a patient with tumor. Therefore, early screening, medication guidance, prognosis determination and relapse monitoring for the patient with tumor can be implemented by detecting a genetic mutation of a circulating tumor DNA (ctDNA) in peripheral blood of the patient. However, a peripheral blood sample has a complex background and low content of ctDNA, and therefore, for the detection of a lowabundance and rare sequence, a detection method and kit having extremely high specificity and sensitivity are required.

At present, existing genetic mutation detection reagents mainly use a TaqMan hydrolysis probe method or an Amplification Refractory Mutation System (ARMS). The TaqMan hydrolysis probe method needs the design of a pair of specific PCR primers and one specific probe, which is complementary to a template. The binding site of the probe is located between the two primers, and a 5'-end and a 3'-end of the probe are respectively marked with a fluorescent group(a donor) and a quenching group (a receptor). When the TaqMan probe is in a complete free state, the fluorescent group and the quenching group are close to each other, the quenching group absorbs excited fluorescence of the fluorescent groupunder the action of excitation light, and Fluorescence Resonance Energy Transfer (FRET) occurs, resulting in that an instrument detects no fluorescence signal. During a PCR amplification process, Taq DNA polymerase uses 5'-3'-exonucleolytic activity to cleave a probe, which is bound to a target sequence, and a fluorescent reporting groupgene and the quenching group become farther away from each other, so as to release a fluorescence signal.

Moreover, the ARMS method uses the characteristic of the DNA polymerase lacking 3'-exonucleolytic activity. If a base at the 3'-end of a primer cans not becomplementarily paired with a target nucleic acid sequence correctly, then the target nucleic acid sequence cannot be effectively amplified. In theory, when performing mutation detection, one universal probe is needed to match a mutant-type primer and a wild-type primer, where the 3'-end of the mutant primer is completely matches mutant-type gene, but does not match wild-type gene. When mutant gene detection is performed, as the mutant-type primer cannot be completely paired with a wild-type template, the elongation of the primer is blocked, so as to realize the detection of mutant gene.

When liquid biopsy is performed with respect to the ctDNA in the peripheral blood of the patient with tumor, as the ctDNA is highly fragmented and the lengths of fragments thereof are distributed between 90 bp and 160 bp, it is found, through previous studies, that the shorter the length of a target sequence, the higher the detection rate of the ctDNA (Anderson et al., 2015). However, there is a problem of a target sequence being excessively long in an existing ctDNA detection technique, regardless of using the TaqMan hydrolysis probe method or the ARMS method. Patent CN 105349654 B discloses a probe, primer, detection system and kit for detecting EGFR gene mutation. The length of a target sequence required for detecting EGFR mutant gene is between 70 bp and 134 bp, where the length of an amplification target sequence required for detecting a mutation site on an exon 18 is between 100 bp and 116 bp, the length of an amplification target sequence required for detecting a mutation site on an exon 19 is between 70 bp and 84 bp, the length of an amplification target sequence required for detecting a mutation site on an exon 20 is between 85 bp and 134 bp, and the length of an amplification target sequence required for detecting a mutation site on an exon 21 is between 109 bp and 121 bp. A greater length of the amplification target sequence inevitably reduces the detection rate of a fragmented ctDNA template, and thus affects the detection sensitivity.

In addition, a mutant-type primer in an existing ARMS method is often incapable of completely blocking the amplification of a non-specific template, and a false positive result is easily generated during detection. Particularly in an end-point detection system, such as a digital PCR system, fluorescence signals of a mutant-type primer and a wild-type primer upon the end-point of amplification are often difficult to distinguish, and thus resulting in insufficient specificity of detection.

According to this, in the field of gene detection, there are requirements for further improving the sensitivity and specificity of nucleic acid detection.

### SUMMARY

For the above-mentioned problems, the present inventor studies the design manners of a primer and a probe on the basis of the ARMS method, so as to complete the present invention.

In an aspect, the present invention provides a combination for detecting nucleic acids, the combination including:
an upstream primer, the 3'-end of which is provided with a target sequence binding region, wherein the 3'-end of the target sequence binding region is capable of being complementary to a specific detection site of a target sequence;
a downstream primer, the 3'-end of which is provided with a target sequence binding region, wherein the 3'-end of the target sequence binding region is also capable of being complementary to the same specific detection site; and
a signal oligonucleotide, wherein the signal oligonucleotide is modified with a first detection group and a second detection group, with the first detection group and the second detection group generating a change in signal by means of a change in distance.

The signal oligonucleotide is designed as a part of the upstream primer and/or the downstream primer, and to be located upstream of the target sequence binding region, or the upstream primer and/or the downstream primer further include a signal detection region upstream of the target sequence binding region, and the signal oligonucleotide is designed to be independent of the upstream primer and/or the downstream primer and have the same sequence as the signal detection region.

The signal detection region is designed to be incapable of being complementarily paired with the target sequence.

By using such design, the present invention has the following beneficial effects:
In an aspect, on the basis of replacing a target-sequence-specific probe with the signal oligonucleotide, the 3'-end of the upstream primer and the downstream primer both matche a certain specific detection site on a target sequence. Through the cooperation of the two methods, the requirement for the length of the target sequence is reduced to a theoretical minimum value,as proved in the examples below, the length of the target sequence does not have to exceed 40 bp. As stated above, in the detection of a highly fragmented nucleic acid sample (for example, ctDNA), a shorter length of a target sequence achieves a higher detection rate, thereby greatly improving the sensitivity of detection.

In another aspect, the combination and reaction system of the invention can greatly avoid a cross reaction. For example, when a mutant-type target nucleic acid sequence is tested, there is no cross reaction with wild-type or other similar or homeologous target nucleic acids. Particularly when a wild-type target nucleic acid sequence and the mutant-type target nucleic acid sequence are tested at the same time, the two have a small cross reaction, so as to better facilitate the detection of rare mutation.

In still another aspect, the combination of the invention can be used for detecting DNA having a short fragment smaller than 100 bp, and can be adaptive to nucleic acid detection of various sample types.

In yet another aspect, the combination of the invention can test the wild-type target nucleic acid sequence and the mutant-type target nucleic acid sequence in one reaction tube at the same time, without needing to perform the qualitative detection or quantitative detection of wild-type gene and mutant-type gene in different tubes, and is particularly suitable for the detection of a rare sample, such as a ctDNA sample of peripheral blood.

In some implementations, primer pairs of the combination of the invention are designed for different types of genetic mutation. When the signal oligonucleotide is designed as a part of a primer, signal oligonucleotide probes corresponding to respective primer pairs may be the same as each other, but the target sequence binding regions thereof are complementary to different mutant-type gene. When the signal oligonucleotide is designed to be present independent of a primer, signal detection regions of respective primer pairs may be the same as each other, but the target sequence binding regions thereof are complementary to different mutant-type gene. Similarly, the combination of the invention can further include multiple additional groups of primer pairs.

Various types of targets are specifically recognized by using the target sequence binding regions of multiple groups of upstream primers and downstream primers, and detection is then performed by the signal oligonucleotide. In multi-detection, for genetic mutation types that do not need typing, in order to realize the detection of different genetic mutation types, it is only necessary to design different upstream primer and downstream primer while the signal oligonucleotide is shared, thereby reducing the use amount of probes. In an aspect, the cost can be reduced, which is conducive to clinical use; in another aspect, the background interference in the reaction system caused by too many probes can also be significantly reduced.

In some implementations, the combination further includes an upstream primer and/or a downstream primer, which can specifically target other nucleic acids. For example, when the upstream primer and the downstream primer of the combination of the invention are designed for mutant-type gene, that is, the 3'-end of the upstream primer and the downstream primer both matche a specific detection site on a target sequence (i.e. a site distinguishing mutant-type gene, which is caused by mutation and is to be detected, from other gene), the mutant-type gene can be amplified. Furthermore, the combination of the invention further includes a wild-type upstream primer, where the 3'-end of the wild-type upstream primer is provided with a site, which matches wild-type gene, so as to be specially annealed with the wild-type gene; and/or a wild-type downstream primer, where the 3'-end of the wild-type downstream primer is provided with a site, which matches the wild-type gene, so as to be specially annealed with the wild-type gene.

In some implementations, the target sequence binding region of the invention can be provided with one to five mismatched bases, which are not complementarily paired with a target sequence. For example, mismatched bases can be provided at the penultimate bit or antepenultimate bit of the 3'-end of the target sequence binding region.

In a first implementation, the combination of the invention includes:
an upstream primer, the 3'-end of which is provided with a target sequence binding region, wherein the 3'-end of the target sequence binding region is capable of being complementary to a specific detection site of a target sequence;
a downstream primer, the 3'-end of which is provided with a target sequence binding region, wherein the 3'-end of the target sequence binding region is also capable of being complementary to the same specific detection site; and
a signal oligonucleotide, wherein the signal oligonucleotide is designed to include a first stem region, a loop region, a second stem region and an anchor region, sequentially from 5' to 3', and is modified with a first detection group and a second detection group, with the first detection group modifying the first stem region, the second detection group modifying the second stem region, a position between the second stem region and the anchor region, or a non-3'-end of the anchor region, and the first detection group and the second detection group generating a change in signal by means of a change in distance.

The upstream primer and/or the downstream primer include a signal detection region upstream of the target sequence binding region, and the signal detection region is designed to be incapable of being complementarily paired with the target sequence.

The first stem region is designed to be partially or completely complementary to the second stem region, and the anchor region is located at the 3'-end of the signal oligonucleotide and is designed to be partially or completely identical to the signal detection region.

It can be understood that, since the anchor region is designed to be completely or partially identical to the signal detection region, the anchor region can be annealed with a complementary sequence of the signal detection region, and can elongate, so as to amplify amplified products of the upstream primer and the downstream primer. In addition, the first stem region is designed to be partially or completely complementary to the second stem region, so as to form a stem part of a stem loop together with the second stem region.

When the combination of the first implementation is used, the first stem region and the second stem region are complementary to each other and form a stem part of a stem loop. When the signal oligonucleotide is of a complete "stem loop" structure, the first detection group and the second detection group are close to each other, and the efficiency of fluorescence resonance energy transfer is higher. When the first stem region of the signal oligonucleotide is hydrolyzed by the 5'-3'-exonucleolytic activity of DNA polymerase, or the "stem loop" structure of the signal oligonucleotide is opened, the first detection group and the second detection group become farther away from each other, and the efficiency of fluorescence resonance energy transfer is reduced, resulting in a change in fluorescence signal, which change is then detected by an instrument.

In some implementations, the signal oligonucleotide of the invention can form a "stem loop" structure, and this stem loop structure still has a stable structure (that is, "the stem loop" is not opened) at annealing temperature of the primer of the invention (for example, up to 75 °C).

In the invention, the length of the first stem region may be 6-20 nt.

In the invention, the length of the second stem region may be 6-20 nt.

In the invention, the loop region may be, for example, a base sequence having a length of 3-25 nt, and may also be, for example, a spacer modification selected from a group consisting of C3, C6, C9 and C12, and may also be, for example, selected from polyethylene glycol.

In the invention, the second stem region and the anchor region may be at an interval of one to five bases.

In a particular implementation, the length of the anchor region may be 15-30 nt.

In an exemplary implementation, the first detection group may be located in the first stem region, and the second detection group may be located in the second stem region; the first detection group may be located in the first stem region, and the second detection group may be located at a non-3'-end of the anchor region; the first detection group may be located in the first stem region, and the second detection group may be located between the second stem region and the anchor region; the second detection group may be located in the first stem region, and the first detection group may be located in the second stem region; the second detection group may be located in the first stem region, and the first detection group may be located at a non-3'-end of the anchor region; or the second detection group may be located in the first stem region, and the first detection group may be located between the second region and the anchor region.

In some implementations, the first detection group or the second detection group may be located at the 5'-end of the first stem region.

In a second implementation, the combination of the invention includes:
an upstream primer, the 3'-end of which is provided with a target sequence binding region, wherein the 3'-end of the target sequence binding region is capable of being complementary to a specific detection site of a target sequence;
a downstream primer, the 3'-end of which is provided with a target sequence binding region, wherein the 3'-end of the target sequence binding region is also capable of being complementary to the same specific detection site; and
a signal oligonucleotide, wherein the signal oligonucleotide is designed as a flexible oligonucleotide, all or part of the sequence of which are the same as that of the signal detection region, and the signal oligonucleotide is modified with a first detection group and a second detection group, with the first detection group and the second detection group generating a change in signal by means of a change in distance, and the first detection group and the second detection group being located at a non-3'-end of the signal oligonucleotide.

The signal detection region is located upstream of the target sequence binding region of the upstream primer and/or the downstream primer, and the signal detection region is designed to be incapable of being complementarily paired with the target sequence.

It can be understood that, since the signal oligonucleotide is designed to be completely or partially identical to the signal detection region, the signal oligonucleotide can be annealed with a complementary sequence of the signal detection region, and can elongate, so as to amplify amplified products of the upstream primer and the downstream primer.

When the combination of the second implementation is used, after the upstream primer and the downstream primer are specially bound to the target sequence and elongate to produce a pre-amplified product, the signal oligonucleotide can be complementarily paired with the pre-amplified product and perform PCR amplification, and the first detection group and the second detection group are integrated into a dual-strand amplified product, which is produced by PCR, such that the distance between the first detection group and the second detection group changes, resulting in a change in fluorescence signal, which change is then detected by an instrument.

In a particular implementation, the length of the signal oligonucleotide may be 15-30 nt.

In an exemplary implementation, the first detection group is located at the 5'-end or a position of any non-3'-end, and the second detection group is spaced apart from the first detection group by 5-25 nt, and is not located at the 3'-end.

In a third implementation, the combination of the invention includes:
an upstream primer, the 3'-end of which is provided with a target sequence binding region, wherein the 3'-end of the target sequence binding region iscapable of being complementary to a specific detection site of a target sequence;
a downstream primer, the 3'-end of which is provided with a target sequence binding region, wherein the 3'-end of the target sequence binding region is also capable of being complementary to the same specific detection site;
a signal oligonucleotide, wherein all or part of the sequence of the signal oligonucleotide are the same as that of the signal detection region, and the signal oligonucleotide is modified with a first detection group and a second detection group, with the first detection group and the second detection group generating a change in signal by means of a change in distance; and
a second primer, wherein all or part of the sequence of the second primer are the same as that of the signal detection region.

The signal detection region is located upstream of the target sequence binding region of the upstream primer and/or the downstream primer, the signal detection region is designed to be incapable of being complementarily paired with a target sequence, and the region on the signal detection region that is the same as the signal oligonucleotide is located downstream of the region thereon that is the same as the second primer.

It can be understood that, in the implementation, the signal oligonucleotide is a TaqMan probe, which can cross pair with a complementary sequence of the signal detection region; and in addition, since the second primer is designed to be completely or partially identical to the signal detection region, the second primer can be annealed with the complementary sequence of the signal detection region, and can elongate, so as to amplify amplified products of the upstream primer and the downstream primer.

When the combination of the third implementation is used, after the upstream primer and the downstream primer are specially bound to the target sequence and elongate to produce a pre-amplified product, the second primer and the signal oligonucleotide can be complementarily paired with the pre-amplified product, and when the second primer elongate, the signal oligonucleotide is hydrolyzed and the first detection group is released, resulting in a change in fluorescence signal, which change is then detected by an instrument.

A person skilled in the art would have been able to design and select a suitable second primer, which only needs to amplify a pre-amplified product of the upstream primer and the downstream primer.

For the signal oligonucleotide in the third implementation of the invention, a person skilled in the art would have been able to design same according to a design principle of a conventional TaqMan hydrolysis probe, as long as a hybridization region is located in a complementary region of the signal detection region.

In a particular implementation, the first detection group and the second detection group are respectively located at the 5'-end of and the 3'-end of the signal oligonucleotide.

In a fourth implementation, the combination of the invention includes:
an upstream primer, the 3'-end of which is provided with a target sequence binding region, wherein the 3'-end of the target sequence binding region is capable of being complementary to a specific detection site of a target sequence;
a downstream primer, the 3'-end of which is provided with a target sequence binding region, wherein the 3'-end of the target sequence binding region is alsocapable of being complementary to the same specific detection site; and
a signal oligonucleotide, wherein the signal oligonucleotide is modified with a first detection group and a second detection group, with the first detection group and the second detection group generating a change in signal by means of a change in distance.

The signal oligonucleotide is designed as a part of the upstream primer and/or the downstream primer, and is located upstream of the target sequence binding region. The signal oligonucleotide includes a first stem region, a loop region and a second stem region, sequentially from 5' to 3'. The first detection group is located in the first stem region, and the second detection group is located in the second stem region.

The first stem region is designed to be partially or completely complementary to the second stem region.

It can be understood that the first stem region is designed to be partially or completely complementary to the second stem region, so as to form a stem part of a stem loop together with the second stem region.

When the combination of the fourth implementation is used, if the signal oligonucleotide is of a complete "stem loop" structure, the first detection group and the second detection group are close to each other, and the efficiency of fluorescence resonance energy transfer is higher. When the first stem region is hydrolyzed by the 5'-3'-exonucleolytic activity of DNA polymerase, or the "stem loop" structure of the signal oligonucleotide is opened, the first detection group and the second detection group become farther away from each other, and the efficiency of fluorescence resonance energy transfer is reduced, resulting in a change in fluorescence signal, which change is then detected by an instrument.

In the invention, a signal oligonucleotide is designed in the upstream primer and/or the downstream primer, such that the use of probes in a traditional ARMS method is further reduced, thereby reducing the cost while the detection performance is satisfied.

In an exemplary implementation, the first detection group may be located in the first stem region, and the second detection group may be located in the second stem region; or the second detection group is located in the first stem region, and the first detection group is located in the second stem region.

In addition, the present invention further provides a method for detecting nucleic acids, the method including the following steps:
mixing the combination of the invention, an amplification reagent and a sample;
amplifying a target sequence, which may be present in the sample;
obtaining a generated change in signal; and
determining, according to the obtained change in signal, whether target nucleic acids are present in the sample.

In a particular implementation, the target nucleic acids are quantified while whether the target nucleic acids are present in the sample is determined.

In some implementations, before the amplification step, the method further includes the step of allocating a sample mixture to different reaction units, and dual-strand nucleic acids are denatured to single-strand nucleic acids. Optionally, before the step of allocating the sample mixture to the different reaction units, the method may further include the step of denaturing dual-strand nucleic acids to single-strand nucleic acids.

In particular, performing determination according to the obtained fluorescence signal refers to detecting whether there is fluorescence resonance energy transfer between the first detection group and the second detection group.

In a particular implementation, amplification refers to amplification by means of PCR, and may include, for example, initial denaturation and a plurality of cycles of denaturation, annealing and elongation.

In a preferred implementation, the template to be tested is ctDNA, and the sample is peripheral blood.

In some implementations, the sample is from a subj ect who has cancer or is suspected to have cancer.

### BRIEF DESCRIPTION OF THE DRAWINGS

Fig. 1 shows a result of detecting point mutation (the mutation when c.2156G>C and p.G719>A) of exon 18 of EGFR gene on a digital PCR platform according to an implementation of the invention;
Fig. 2 shows linear regression analysis results of quantitative data of a high-value sample, a medium-value sample and a low-value sample in solutions having different fragment lengths in Comparative Example 1;
Fig. 3 shows a result of detecting point mutation (the mutation when c.2156G>C and p.G719>A) of exon 18 of EGFR gene on a Q-PCR test platform according to another implementation of the invention;
Fig. 4 shows a result of detecting point mutation (c.2369C>T and p.T790>M) of EGFR gene on a digital PCR platform according to still another implementation of the invention; and
Fig. 5 shows linear regression analysis results of quantitative data of a high-value sample and a low-value sample in solutions having different fragment lengths in Comparative Example 2.

### DETAILED DESCRIPTION

The invention will be specifically illustrated below in conjunction with the particular implementations and examples, and the advantages and various effects of the invention will thus be presented more clearly. A person skilled in the art should understand that those particular implementations and examples are used for describe the invention, rather than limiting the invention. All other implementations obtained by a person of ordinary skill in the art on the basis of the implementations of the invention without any inventive effort shall fall within the scope of protection of the invention.

Throughout the description, unless otherwise specifically stated, the terms used herein should be understood as the meaning commonly used in the art. Therefore, unless otherwise defined, all technological and scientific terms used herein have the same meaning as commonly understood by a person of ordinary skill in the art of the invention. If a contradiction occurs, the description is taken as priority.

The term of "nucleic acid" herein refers to a single-strand and/or dual-strand polymer of nucleotide monomers, and includes, but not limited to 2'-deoxyribonucleic acid (DNA) and ribonucleic acid (RNA), each of which is connected by means of phosphodiester bonds between nucleotide or analogues between nucleotide. The nucleotide monomer in nucleic acids may be referred to as a "nucleotide residue". The nucleic acids can be completely formed of deoxyribonucleic acids, completely formed of ribonucleic acids or formed of a chimeric mixture thereof, and can include an analogue of a nucleotide. The nucleotide monomer unit can include any one of the nucleotides as stated herein, and includes, but not limited to a nucleotide, and/or an analogues of a nucleotide (for example, a modified nucleotide). The size of a nucleic acid generally ranges from several nucleotide residues to thousands of nucleotide residues. The term of "oligonucleotide" generally refers to a nucleotide polymer having a relatively short length (for example, less than 80). Unless otherwise pointed out, it should be understood that, every time a nucleic acid sequence is presented, the nucleic acid, from left to right, is in an order from 5' to 3'. Unless otherwise pointed out, "A"refers to adenine, "C" refers to cytosine, "G" refers to guanine, "T" refers to thymine, and "U" refers to uracil.

According to customary terms in the art, the length of the nucleic acid can be represented by base pair (abbreviated as "bp"), nucleotide (abbreviated as "nt") or kilobase (abbreviated as "kb").

The term of "base complementary pairing" herein refers to the correlation between A and T, between A and U and between G and C is mutual connection by means of hydrogen bonds. Correspondingly, "mismatched bases" refer to all the pairing situations other than those specified in "base complementary pairing", for example, A is paired with C, A is paired with G, T is paired with G, or T is paired with C.

As stated above, the target sequence binding region of the invention can be provided with one to five mismatched bases, and the mismatched bases and bases at the 3'-end can jointly function, such that the amplification rate of a primer in a template that is not complementary to the primer at the 3'-end thereof is significantly reduced. The configuration of such mismatched bases falls within the scope of capability of a person skilled in the art. In some implementations, mismatch is introduced to the antepenultimate bit of the upstream primer and/or the downstream primer.

In some implementation, the method of the invention is to detect nucleic acid mutation.

In the invention, "mutation" may be selected from the following items: base substitution mutation, insertion mutation and deletion mutation. In an exemplary implementation, the mutation is base substitution mutation, that is, two types of target sequences (a wild-type target sequence and a mutant-type target sequence) to be detected have no difference in base count, but are different in the type of one or more bases.

In some implementation, the mutation of the invention is single-nucleotide mutation.

In the invention, the term of "specific detection site" refers to a base site that can distinguish a target nucleic acid from other nucleic acids. For example, for a point mutation situation, if there is a mutant base between a wild-type nucleic acid and a mutant-type nucleic acid or between different mutant-type nucleic acids, then the "specific detection site" is the mutant base; and for a nucleic acid typing situation (for example, performing typing on some viruses), similar to mutation situation, if there is one or several base differences between different types of nucleic acids, then the "specific detection site" is one of the different bases.

The expression of "the 3'-end of the upstream primer and the downstream primer both can be complementary to a specific detection site of a target sequence" refers to the 3'-endes of the upstream primer and the downstream primer both can be complementary to the same specific detection site of a target sequence. For example, for a point mutation situation, the 3'-end of the upstream primer and the downstream primer in the combination of the invention are complementary to the mutant base; and for a nucleic acid typing situation, the 3'-end of the upstream primer in the combination of the invention is complementary to a different base, and the 3'-end of the downstream primer is also complementary to the different base. By using such design, when the 3'-end of a primer is not complementary to a specific detection site, the elongation of the primer is blocked, so as to realize the detection of a target sequence.

The term of "primer" herein represents such a oligonucleotide as follows: the oligonucleotide "initiates" DNA synthesis by means of a template-dependent DNA polymerase, for example, the 3'-end of the oligonucleotide provides a free 3'-OH base, and more "nucleotides" can be connected to the 3'-OH base by means of the template-dependent DNA polymerase, so as to establish 3'-5'-phosphodiester bonds, such that deoxyribonucleoside triphosphate is used, and thus pyrophosphoric acids are released.

The terms of "target sequence", "target nucleic acid", "target nucleic acid sequence" or "target" can be used interchangeably herein, and refer to a nucleic acid sequence part to be amplified, to be detected, or to be amplified and detected. The nucleic acid sequence part can be annealed or hybridized with a probe or primer under a hybridization, annealing or amplification condition.

The term of "hybridization" represents bases of two nucleic acids are paired with each other, which leads to the formation of a dual-strand structure. It can be understood that they do not have to be hundred-percent complementary to each other along the overall length to realize hybridization.

The term of "upstream primer" herein, also known as forward primer, is an oligonucleotide that continuously elongates along a negative strand. The term of "downstream primer", also known as reverse primer, as used in the invention is an oligonucleotide that continuously elongates along a positive strand. The positive strand is a sense strand or a positive-sense strand, and is also referred to as a coding strand. The positive strand is generally located at the upper end of a double stranded DNA, the direction thereof from left to right is 5' to 3', and a base sequence of the positive strand is basically the same as mRNA of the gene. A primer that is bound to the strand is a reverse primer. The negative strand includes a nonsense strand, is also referred to as a non-coding strand. The negative strand is complementary to the positive strand, and a primer that is bound to the negative strand is a forward primer. It should be understood that, when the designations of the positive-sense strand and the antisense strand interchange, the names of the corresponding forward primer and reverse primer may also interchange along with same.

In context of describing a nucleic acid sequence, the expressions of "upstream of', "located upstream of...", "there are ... upstream", etc. herein each represent a part of a nucleic acid sequence that is closer to the 5'-end than a region, which is referred to, on the same nucleic acid sequence. For example, the part may be closely adjacent to the region referred to, and may also be spaced apart from the region referred to by one or more bases. Correspondingly, in the context of describing a nucleic acid sequence, the terms of "downstream", "located downstream of...", "there are ... downstream", etc. used herein each represent a part of a nucleic acid sequence that is closer to the 3'-end than a region, which is referred to, on the same nucleic acid sequence. For example, the part may be closely adjacent to the region referred to, and may also be spaced apart from the region referred to by one or more bases. It should be understood that, when the nucleic acid described is a double stranded nucleic acid, the representations of "upstream" and "downstream" often take the 5'-end and 3'-end of the positive-sense strand as a reference, unless otherwise specified.

The terms of "TaqMan probe" and "hydrolysis probe" can be used interchangeably herein. The TaqMan probe is a fluorescence detection technique that is developed from a real-time PCR technology platform, with the 5'-end of the probe containing a first detection group, and the 3'-end thereof containing a second detection group. When the probe is complete, a fluorescence signal emitted by the first detection group is received by the second detection group. When PCR amplification is performed, the probe is enzyme cleaved and degraded by the exonuclease activity from the 5'-end to the 3'-end of Taq DNA polymerase, such that the first detection group and the second detection group are separated from each other, and a fluorescence signal is emitted, thereby realizing complete synchronization of fluorescence signal accumulation and PCR product formation.

The expression of "stringent condition" herein may be any one of a low-stringent condition, a medium-stringent condition and a high-stringent condition. The "low-stringent condition" is, for example, a condition involving 5x SSC solution, 5x Denhardt solution, 0.5-percent SDS, 50-percent formamide and 32°C. In addition, the "medium-stringent condition" is, for example, a condition involving 5x SSC solution, 5x Denhardt solution, 0.5-percent SDS, 50-percent formamide and 42°C, and the "high-stringent condition" is, for example, a condition involving 5x SSC solution, 5x Denhardt solution, 0.5-percent SDS, 50-percent formamide and 50°C. Under these conditions, it is expected that the higher the temperature, the more effectively can high-homology polynucleotides (for example, DNA) be obtained. Although there are various factors affecting the hybridization stringency, such as temperature, probe concentration, probe length, ion strength, time and salt concentration, a person skilled in the art would have been able to arrive at similar stringency by means of appropriately selecting these factors.

The term of "flexible oligonucleotide" herein refers to an oligonucleotide when in a single-strand state being curled due to molecular flexibility, and after being hybridized or being annealed and elongating to become in a dual-strand state, the oligonucleotide being fixed as a relatively rigid double-helix structure due to the action of a hydrogen bond, such that the distance between a first detection group and a second detection group which are marked on the flexible oligonucleotide changes, so as to generate a detectable signal. For example, a flexible oligonucleotide can be obtained with reference to the disclosure of US 9845492 B2.

In some implementations, one of the first detection group and the second detection group of the invention may be a fluorescent group, and the other one may be a quenching group, or other group that cans generate a change in signal with the fluorescent group by means of fluorescence resonance energy transfer.

In an exemplary implementation, the group that generate a change in signal by means of fluorescence resonance energy transfer may be Cy3 or Cy5, since they can generate a change in fluorescence signal by means of the action of FRET.

In the invention, the fluorescent group may be, for example, selected from a group consisting of the following items: FAM, HEX, VIC, ROX, Cy3, Cy5 and Cy5.5.

In the invention, the quenching group may be, for example, selected from a group consisting of the following items: TAMRA, BHQ1, BHQ2, BHQ3, DABCYL, QXL and DDQI.

In some embodiments, the signal oligonucleotide and a pre-amplified product of the upstream primer and the downstream primer are annealed and elongate, and the first detection group and the second detection group are integrated into a double-strand amplified product that is produced by elongation, so as to generate a change (an increase) in distance.

In some implementations, the combination of the invention may be a kit, the kit further including an amplification reagent.

The term of "amplification reagent" in the invention refers to a reagent for a PCR, including, but not limited to dNTP, DNA polymerase, and some reagents that promote the PCR, for example, KCl, MgCl₂, Tris-HCl and dithiothreitol (DTT).

In the invention, the combination and various components in the kit may be present in a separately packaged form, or may be present in a pre-mixed form.

In the invention, the purposes of "initial denaturation" and "denaturation" are to break hydrogen bonds between paired complementary bases of a double stranded DNA, so as to allow double strands to be separated into two individual strands. For example, individual strands can be formed by means of heating a mixture that contains double stranded DNA, for example, the mixture is heated to 90°C, 92°C, 95°C or 98°C to dissociate the double stranded DNA. After the dissociation is completed, the mixture is cooled to the room temperature or below the room temperature. For another example, the individual strands can also be formed by breaking the hydrogen bonds between the double stranded DNA by means of changing the ion strength of a solution (for example, adding acids, alkali, salt, etc.), and the dissociation of the double stranded DNA into single stranded DNA can also be realized by using an enzyme (for example, a helicase). In addition, in a reaction involving a hot start polymerase, the step of "initial denaturation" further includes performing heat activation on the polymerase. In the case where a digital PCR platform is involved, after the double stranded DNA is denatured to single stranded DNA, "initial denaturation" further includes allocating the individual strands to droplets/micropores, so as to increase the number of effective targets that participant in the reaction, thereby improving the reaction sensitivity.

In some implementations, the amplification can be divided into two cyclic amplification stages. The first cyclic amplification stage may have 3-15 cycles, and the second cyclic amplification stage may have 30-50 cycles.

The program applicable to amplification and common reaction conditions for amplification are well known to a person skilled in the art. In some exemplary implementations, the specific reaction condition may be: performing initial denaturation at 92°C to 96°C for 5-15 minutes; performing denaturation at 92°C to 96°C for 10-60 seconds, and performing annealing at 55°C to 75°C and elongation for 30-90 seconds, and implementing 3-15 cycles in total; performing denaturation at 92°C to 96°C for 10-60 seconds, performing annealing at 45°C to 65°C and elongation for 30-90 seconds, and implementing 35-50 cycles; and terminating the reaction at 4°C to 15°C. Optionally, after the step of heat cycles, the method may further include an inactivation step, for example, at 94°C to 98°C for 5-15 minutes. In some other exemplary implementations, the amplification includes: performing initial denaturation at 90°C to 96°C for 5-15 minutes; performing denaturation at 90°C to 95°C for 10-60 seconds, and performing annealing at 50°C to 75°C and elongation for 30-90 seconds, and implementing 35-50 cycles; and terminating the reaction at 4°C to 15°C. Optionally, after the step of heat cycles, the method may further include an inactivation step, for example, at 94°C to 98°C for 5-15 minutes. In a particular implementation, the amplification includes: performing initial denaturation at 95°C for 10 minutes; performing denaturation at 94°C for 30 seconds, and performing annealing at 55°C and elongation for 60 seconds, and implementing 45 cycles in total; and terminating the reaction at 10°C.

In the case of only being used for performing pre-amplification on a target sequence in a sample, the concentration of the upstream primer or the downstream primer in the reaction system may be, for example, 15 nM to 150 nM, preferably be 30 nM to 90 nM, and more preferably be 45 nM; otherwise, the concentration of the upstream primer or the downstream primer in the reaction system may be 150 nM to 1800 nM, preferably be 300 nM to 900 nM, and more preferably be 450 nM.

When a signal oligonucleotide is present independently, the concentration thereof in the reaction system may be, for example, 150 nM to 1500 nM, preferably be 300 nM to 600 nM, and more preferably be 450 nM.

In the invention, the expressions of "first", "second", etc. are merely for descriptive purpose and used for distinguishing defined substances, and do not define the order or precedence in any manner.

In the invention, a sample under test may be selected from a group consisting of a free peripheral blood sample, other free nucleic acid sample from a human or a microorganism, a Formalin fixed and paraffin embedded tissue (FFPE) sample, a fresh tissue sample, a urine sample, a lavage fluid sample, a cerebrospinal fluid sample, a cultured cell line sample and an artificially synthesized plasmid sample and a combination thereof.

The invention will be illustrated in more details by means of particular examples, but the invention is not limited to the examples.

### Example 1: Detect point mutation of c.2156G>C and p.G719>A of exon 18 of EGFR gene on a ddPCR platform

### Preparation of a sample:

The QIAamp DNA Mini and Blood Mini Kit from QIAGEN Company is used according to an operation instruction for the kit to extract HEK-293T cell DNA, the HEK-293T cell DNA and G719A plasmid DNA, which is provided by SHANGHAI SANGON BIOTECH, are respectively subjected to sonication, and screening purification through magnetic beads, so as to obtain a wild-type template (HEK-293T cell line DNA) and a mutant-type template (G719A plasmid DNA). The two types of templates are mixed according to a certain ratio, so as to obtain a simulated clinical sample containing G719A point mutation of EGFR gene. Moreover, a fragmented wild-type DNA is used to prepare a negative control (Neg), and a TE Buffer is used as a no template control (NTC).

The design of primers and a signal oligonucleotide: an upstream primer, a downstream primer and a signal oligonucleotide are designed for point mutation at EGFR exon 18, and the sequences thereof are as shown in Table 1.

**Table 1**

| Name | Number | Nucleotide sequence (5'-3') | Modification |
|---|---|---|---|
| Mutant-type upstream primer | SEQ ID NO:1 | CCGACTGCTGACAAAGTATAAAAAA GATCAAAGTGCTGGC | |
| Mutant-type downstream primer | SEQ ID NO:2 | GCCGAACGCACCGGAGG | |
| Signal oligonucleotide | SEQ ID NO:3 | CCGCCTGT-C9- ACAGGCGGTACCGACTGCTGACAAA GTAT | 5'FAM, BHQ1 (17), C9 spacer (8) |

The mutant-type upstream primer (SEQ ID NO: 1) has a total length of 40 bp, the first base to the 20th base of the 3'-end thereof is a target sequence binding region, and the first base to the 20th base of the 5'-end thereof are the same as the first base to the 20th base of the 3'-end of the signal oligonucleotide (SEQ ID NO:3). The mutant-type downstream primer (SEQ ID NO:2) has a total length of 17 bp, and is bound to a target sequence. The signal oligonucleotide (SEQ ID NO:3) has a total length of 37 bp, the first base to the 20th base of the 3'-end thereof is an anchor region, the eight bases of the 5'-end thereof is a first stem region thereof, and the 9th base to the 16th base of the 5'-end thereof is a second stem region thereof.

The reaction system is formulated as shown in Table 2 below.

**Table 2**

| Reagent component | Concentration |
|---|---|
| 2x ddPCR Supermix for Probes | 1x |
| Mutant-type upstream primer | 45 nM |
| Mutant-type downstream primer | 450 nM |
| Signal oligonucleotide | 450 nM |
| DNA Sample | 15 ng |
| Ultrapure water | Add ultrapure water to reach 20 µL |

After formulation is completed, 20 µL of a reaction solution that contains a template to be detected is placed in a metal bath, denaturation is performed at 95°C for one minute, and the reaction solution is then immediately placed into a refrigerator at 2°C to 8°C to be cooled for 2-3 minutes.

The 20 µL of the cooled PCR reaction solution is added into sample pores of a droplet generator cartridge, 70 µL of droplet generator oil (Bio-Rad, 1863005) is then added into oil pores of the droplet generator cartridge, and finally, the droplet generator cartridge is sealed by using a sealing strip (Bio-Rad, 1863009).

The prepared droplet generator cartridge is placed into a droplet generator, so as to start the generation of droplets. After approximate 2 minutes, the preparation of the droplets is completed, the cartridge is taken out, and 40 µL of droplets are carefully transferred from the uppermost row of pores to a 96-pore PCR plate (Bio-Rad, 1200192).

### The read of amplification:

After film sealing processing is performed on the 96-pore plate, the 96-pore plate is placed in a PCR thermal cycler (Bio-Rad, PX1 PCR Plate Sealer), and specific enrichment, specific amplification of an enriched template, and detection of a fluorescence signal are sequentially performed on a target nucleic acid sequence. The reaction program involves: performing initial denaturation at 95°C for 10 minutes; performing denaturation at 94°C for 30 seconds, and performing annealing at 65°C and elongation for 60 seconds, and implementing 45 cycles in total; performing inactivation at 98°C for 10 minutes; and terminating the reaction at 10°C.

During the reaction process, at first cycles of the PCR, the mutant-type upstream primer and the mutant-type downstream primer specially amplify the target nucleic acid sequence. After amplification, a sequence that is complementary to a "signal detection region" of the upstream primer will be newly added to be subsequently paired with and recognized by the signal oligonucleotide.

After the reaction is completed, the signal oligonucleotide and the mutant-type downstream primer can be paired with a corresponding enriched template and amplify same, and when the mutant-type downstream primer is amplified to the anchor region, the first stem region is hydrolyzed by a DNA polymerase using the 5'-3'-exonucleolytic activity thereof, such that a fluorescent group is separated from a quenching group, and a fluorescence signal is emitted, so as to detect the mutant-type template.

### The analysis of data:

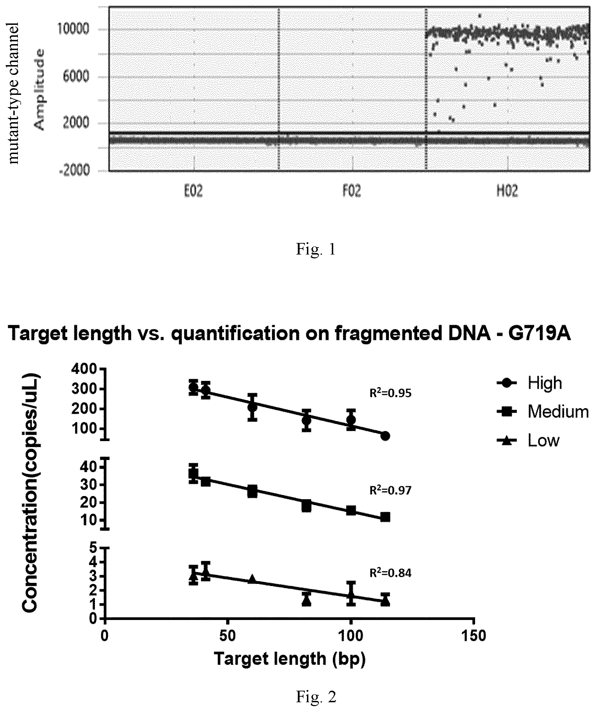

Data analysis is performed by using Quantasoft digital PCR analysis software from BIO-RAD COMPANY, and a result is as shown in Fig. 1. "E02" is a test result of the no template control (NTC), "F02"is a test result of the negative control (Neg), and "H02" is a result of the simulated clinical sample. It can be seen from Fig. 1 that there is no non-specific amplification in both the negative control and the no template control, and mutation of c.2156G>C and p.G719>A can be specially recognized from the simulated clinical sample.

### Comparative example 1

In the Example 1, the upstream primer and the downstream primer both specially recognize point mutation of exon 18 of EGFR gene. In order to investigate the detection effect difference between the solution of two primers both specially recognizing mutant gene and the solution of only one primer specially recognizing mutant gene, the following experiment is conducted.

### Preparation of a sample:

The preparation process of the wild-type template (HEK-293T cell line DNA) and the mutant-type template (G719A plasmid DNA) is identical to that in Example 1. The two types of templates are mixed according to different ratios, so as to obtain a high-value sample (the theoretical concentration being 393 copies/µL), a medium-value sample (the theoretical concentration being 44 copies/µL) and a low-value sample (the theoretical concentration being 4 copies/µL), each of which contains G719A point mutation of EGFR gene. Moreover, a fragmented wild-type DNA is used to prepare a negative control (Neg), and a TE Buffer is used as a no template control (NTC).

The design of primers and a signal oligonucleotide:
The solution of two primers both specially recognizing mutant gene uses the design in Table 1, while the solution of a single primer specially recognizing mutant gene uses the design in Table 3.

**Table 3**

| Name of primer | Number | Nucleotide sequence (5'-3') | modification |
|---|---|---|---|
| Mutant-type downstream primer | SEQ ID NO:2 | GCCGAACGCACCGGAGG | |
| signal oligonucleotide | SEQ ID NOT | CCGCCTGT-C9- ACAGGCGGTACCGACTGCTGACAAA GTAT | 5'FAM, BHQ1 (17), C9 spacer (8) |
| Upstream primer 1 | SEQ ID NO:7 | CCGACTGCTGACAAAGTATAATTCAA AAAGATCAAAGTGCTG | |
| Upstream primer 2 | SEQ ID NO:8 | CCGACTGCTGACAAAGTATAGGATCT TGAAGGAAACTGA | |
| Upstream primer 3 | SEQ ID NO:9 | CCGACTGCTGACAAAGTATAGCTCCC AACCAAACTCTCTTG | |
| Upstream primer 4 | SEQ ID NO: 10 | CCGACTGCTGACAAAGTATATCTTAC ACCCAGTGGAGAAG | |
| Upstream primer 5 | SEQ ID NO:11 | CCGACTGCTGACAAAGTATACAGCTT GTGGAGCTTCTTACA | |

The downstream primer and the signal oligonucleotide in Table 3 are the same as those in Table 1. In addition, a group of upstream primers are designed, and these upstream primers do not specially recognize point mutation.

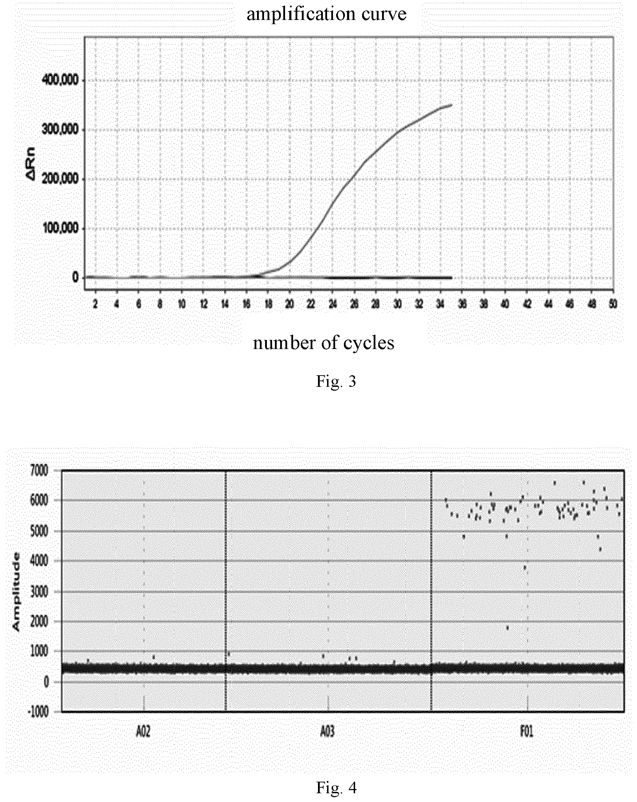

The two solutions use the same mutant-type downstream primer and different mutant-type upstream primers/upstream primers, such that corresponding target sequences thereof are different in length. For details, see Fig. 4.

**Table 4**

| Name of primer | Number | Length of corresponding target sequence |
|---|---|---|
| Mutant-type upstream primer | SEQ ID NO: 1 | 36 bp |
| Upstream primer 1 | SEQ ID NO:7 | 41 bp |
| Upstream primer 2 | SEQ ID NO:8 | 60 bp |
| Upstream primer 3 | SEQ ID NO:9 | 82 bp |
| Upstream primer 4 | SEQ ID NO: 10 | 100 bp |
| Upstream primer 5 | SEQ ID NO:11 | 114 bp |

The reaction system is formulated as shown in Table 5 below.

**Table 5**

| Reagent | Concentration |
|---|---|
| 2x ddPCR Supermix for Probes | 1x |
| Mutant-type upstream primer or upstream primer 1/2/3/4/5 | 45 nM |
| Mutant-type downstream primer | 450 nM |
| Signal oligonucleotide | 450 nM |
| DNA sample | 15 ng |
| Ultrapure water | Add ultrapure water to reach 20 µL |

Before the droplets are generated, 20 µL of a formulated reaction solution that contains a template under test is placed in a metal bath, and after denaturation is performed at 95°C for one minute, the reaction solution is then immediately placed into a refrigerator at 2°C to 8°C for 2-3 minutes.

The 20 µL of the cooled PCR reaction solution is added into sample pores of a droplet generator cartridge, 70 µL of droplet generator oil is then added into oil pores of the droplet generator cartridge, and finally, the droplet generator cartridge is sealed by using a sealing strip.

The prepared droplet generator cartridge is placed into a droplet generator, so as to start the generation of droplets. After approximate 2 minutes, the preparation of the droplets is completed, the cartridge is taken out, and 40 µL of droplets are carefully transferred from the uppermost row of pores to a 96-pore PCR plate.

### The read of amplification:

After film sealing processing is performed on the 96-pore plate, the 96-pore plate is placed in a thermal cycler for PCR amplification. The used program involves: performing initial denaturation at 95°C for 10 minutes; performing denaturation at 94°C for 30 seconds, and performing annealing at 55°C and elongation for 60 seconds, and implementing 48 cycles in total; performing inactivation at 98°C for 10 minutes; and terminating the reaction at 10°C.

After PCR amplification ends, the 96-pore plate is placed in a droplet analyzer, and an FAM/HEX channel is selected to perform signal reading.

### Statistical analysis:

The QuantaSoft analysis software is used to analyze the strength and number of fluorescence signals to obtain the copy number and concentration of the mutant type of point mutation of exon 18 of EGFR gene, and the results are as shown in Table 6.

**Table 6**

| | Quantitative mean (unit: copies/µL) | | |
|---|---|---|---|
| Length of fragment | High-value sample | Medium-value sample | Low-value sample |
| 36 bp | 308.1 | 36.5 | 3.1 |
| 41 bp | 293.5 | 31.9 | 3.4 |
| 60 bp | 207.7 | 26.3 | 2.8 |
| 82 bp | 141.9 | 18.2 | 1.4 |
| 100 bp | 145.5 | 15.6 | 1.8 |
| 114 bp | 64.4 | 11.9 | 1.4 |

It can be seen from Table 6 that the shorter the fragment of a target sequence, the higher the capability of sample quantification, that is, the higher the capability of detecting a fragment of a G719A mutant type.

Linear regression analysis is performed on the data in Table 6, and an analysis result is as shown in Fig. 2. It can be seen that, when a mutant-type high-value sample, medium-value sample and low-value sample are detected, the detection capability is negatively correlated with the length of the target sequence. The shorter the target sequence, the more the mutant-type fragments are detected, and the higher the detection capability; otherwise, the lower the detection capability.

### Example 2: Detect G719A point mutation of exon 18 of EGFR gene on a Q-PCR platform

### Preparation of a sample:

The QIAamp DNA Mini and Blood Mini Kit from QIAGEN Company is used according to an operation instruction for the kit to extract HEK-293T cell DNA, the HEK-293T cell DNA and G719A plasmid DNA, which is provided by SHANGHAI SANGON BIOTECH, are respectively subjected to sonication, and screening purification through magnetic beads, so as to obtain a wild-type template (HEK-293T cell line DNA) and a mutant-type template (G719A plasmid DNA). The two types of templates are mixed according to a certain ratio, so as to obtain a simulated clinical sample containing G719A point mutation of EGFR gene. Moreover, a fragmented wild-type DNA is used to prepare a negative control (Neg), and a TE Buffer is used as a no template control (NTC).

### The formulation for a reaction:

The design of primers and a signal oligonucleotide: an upstream primer, a downstream primer and a signal oligonucleotide are designed for point mutation at EGFR exon 18, and the sequences thereof are as shown in Table 7.

**Table 7**

| Name | Number | Nucleotide sequence (5'-3') | Modification |
|---|---|---|---|
| Downstream primer | SEQ ID NO:2 | CAAAGCAGAAACTCACATCG | |
| Upstream primer | SEQ ID NO:4 | CGACGGCTGACACGATAACTCTGA CTCACCACAACGAGAATTCAAAAA GATCAAAGTGCTGGC | |
| Second primer | SEQ ID NO:5 | CGACGGCTGACACGATA | |
| Signal oligonucleotide | SEQ ID NO:6 | CTCTGACTCACCACAACG | 5 TAM 3'BHQ1 |

The reaction system is formulated as shown in Table 8 below.

**Table 8**

| Reagent component | Concentration |
|---|---|
| MgCl₂ | 3.8 mM |
| Taq enzyme | 2U |
| dNTP | 0.8 mM |
| Upstream primer | 45 nM |
| Second primer | 450 nM |
| Downstream primer | 450 nM |
| Signal oligonucleotide | 450 nM |
| DNA sample | 15 ng |
| Ultrapure water | Add ultrapure water to reach 20 µL |

### The read of amplification:

A sample is lightly mixed uniformly after a PCR tube is covered and sealed, and then, after the sample is momentarily centrifuged, the sample is placed in the room temperature to stand for five minutes. The PCR tube is placed in a handheld centrifuge again, and is transferred into a tray of a fluorescence quantitative PCR instrument (an ABI 7500 real-time fluorescence quantitative PCR system) after being momentarily centrifuged. The used program involves: performing initial denaturation at 95°C for 5 minutes; performing denaturation at 94°C for 30 seconds, and performing annealing at 60°C and elongation for 30 seconds, and implementing 10 cycles in total without illumination; performing annealing at 56°C and elongation for 30 seconds, and implementing 30 cycles in total with illumination; and terminating the reaction at 10°C.

### The analysis of data:

Data analysis is performed by using a Q-PCR test platform, and a result are as shown in Fig. 3. It can be seen from Fig. 3 that there is no non-specific amplification in both the negative control and the no template control, and a G719A fragment can be specially recognized from the simulated clinical sample.

### Example 3: Detect point mutation of c.2369C>C and p.G719>A of EGFR gene on a ddPCR platform

### Preparation of a sample:

The QIAamp DNA Mini and Blood Mini Kit from QIAGEN Company is used according to an operation instruction for the kit to extract HEK-293T cell DNA and NCI-H1975 cell line DNA, the two types of cell line DNA are respectively subj ected to sonication, and screening purification through magnetic beads, so as to obtain a wild-type EGFR template (HEK-293T cell line DNA) and a mutant-type EGFR T790M template (NCI-H1975 cell line DNA). The two types of templates are mixed according to a certain ratio, so as to obtain a simulated clinical sample containing T790M point mutation of EGFR gene. Moreover, a fragmented wild-type DNA is used to prepare a negative control (Neg), and a TE Buffer is used as a no template control (NTC).

The sequences of the primers are as shown in Table 9.

**Table 9**

| Name of primer | Number | Nucleotide sequence (5'-3') | Modification |
|---|---|---|---|
| Mutant-type upstream primer | SEQ ID NO:12 | | 5'FAM, C9(7), BHQ1(15) |
| Wild-type upstream primer (blocking oligonucleotide) | SEQ ID NO:13 | CACCGTGCARCTCATCAC | 3' spacer C3 |
| Mutant-type downstream primer | SEQ ID NO:14 | CAGCCGAAGGGCATGAGCTGCA | |

The reaction system is formulated as shown in Table 10 below.

**Table 10**

| Reagent component | Concentration |
|---|---|
| 2x ddPCR Supermix for Probes | 1x |
| Mutant-type upstream primer | 360 nM |
| Wild-type upstream primer | 360 nM |
| Mutant-type downstream primer | 360 nM |
| DNA sample | 15 ng |
| Ultrapure water | Add ultrapure water to reach 20 µL |

After formulation is completed, 20 µL of a reaction solution that contains a template under test is placed in a metal bath, denaturation is performed at 95°C for one minute, and the reaction solution is then immediately placed into a refrigerator at 2°C to 8°C to be cooled for 2-3 minutes.

The 20 µL of the cooled PCR reaction solution is added into sample pores of a droplet generator cartridge, 70 µL of droplet generator oil is then added into oil pores of the droplet generator cartridge, and finally, the droplet generator cartridge is sealed by using a sealing strip.

The prepared droplet generator cartridge is placed into a droplet generator, so as to start the generation of droplets. After approximate 2 minutes, the preparation of the droplets is completed, the cartridge is taken out, and 40 µL of droplets are carefully transferred from the uppermost row of pores to a 96-pore PCR plate.

### The read of amplification:

After film sealing processing is performed on the 96-pore plate, the 96-pore plate is placed in a thermal cycler for PCR amplification. The used program involves: performing initial denaturation at 95°C for 10 minutes; performing denaturation at 94°C for 30 seconds, and performing annealing at 56°C and elongation for 60 seconds, and implementing 48 cycles in total; performing inactivation at 98°C for 10 minutes; and terminating the reaction at 10°C.

After PCR amplification ends, the 96-pore plate is placed in a droplet analyzer, and an FAM/HEX channel is selected to perform signal reading.

### The analysis of data:

Data analysis is performed by using Quantasoft digital PCR analysis software from BIO-RAD COMPANY, and a result is as shown in Fig. 4. "A02" is a test result of the no template control (NTC), "A03"is a test result of the negative control (Neg), and "F01" is a result of the simulated clinical sample. In the solution of the invention, there is no non-specific amplification in both the negative control and the no template control in the ddPCR test platform, and a T790M fragment can be specially recognized from an H1975 sample.

### Comparative example 2

In the Example 3, the upstream primer and the downstream primer both specially recognize point mutation at exon 20 of EGFR gene. In the solution of the invention, both the mutant-type F and the mutant-type R specially recognize the mutation site. In order to investigate the detection effect difference between the solution of two primers both specially recognizing mutant gene and the solution of only one primer specially recognizing mutant gene, the following experiment is conducted. Preparation of a sample:
The preparation process of the wild-type template (HEK-293T cell line DNA) and the mutant-type template (NCI-H1975 cell line DNA) is identical to that in Example 3. The two types of templates are mixed according to different ratios, so as to obtain a high-value sample (the theoretical concentration being 150 copies/µL) and a low-value sample (the theoretical concentration being 15 copies/µL), each of which contains T790M point mutation of EGFR gene. Moreover, a fragmented wild-type DNA is used to prepare a negative control (Neg), and a TE Buffer is used as a no template control (NTC).

### The design of primers:

The solution of two primers both specially recognizing point mutation uses the design in Table 10, while the solution of a single primer specially recognizing point mutation uses the design in Table 11.

**Table 11**

| Name of primer | Number | Nucleotide sequence (5'-3') | Modification |
|---|---|---|---|
| Mutant-type upstream primer | SEQ ID NO:12 | | 5'FAM, C9(7), BHQ1(15) |
| Wild-type upstream primer (blocking oligonucleotide) | SEQ ID NO:13 | CACCGTGCARCTCATCAC | 3' spacer C3 |
| Downstream primer 1 | SEQ ID NO:15 | CCCGGACATAGTCCAGGA | |
| Downstream primer 2 | SEQ ID NO:16 | TGAGCAGGTACTGGGAGC | |
| Downstream primer 3 | SEQ ID NO:17 | CTGATTACCTTTGCGATC | |

The mutant-type upstream primer and the wild-type upstream primer in Table 11 are the same as those in Table 10. In addition, a group of downstream primers are designed, and these downstream primers do not specially recognize point mutation.

The two solutions use the same mutant-type upstream primer and wild-type upstream primer, and different mutant-type downstream primers/downstream primers, such that corresponding target sequences thereof are different in length. For details, see Table 12.

**Table 12**

| Name of primer | Number | Length of corresponding target sequence |
|---|---|---|
| Mutant-type downstream primer | SEQ ID NO:14 | 41 bp |
| Downstream primer 1 | SEQ ID NO:15 | 61 bp |
| Downstream primer 2 | SEQ ID NO:16 | 97 bp |
| Downstream primer 3 | SEQ ID NO:17 | 128 bp |

The reaction system is formulated as shown in Table 13 below.

**Table 13**

| Reagent | Concentration |
|---|---|
| 2x ddPCR Supermix for Probes | 1x |
| Mutant-type upstream primer | 360 nM |
| Wild-type upstream primer | 360 nM |
| Mutant-type downstream primer or downstream primer 1/2/3 | 360 nM |
| DNA sample | 15 ng |
| Ultrapure water | Add ultrapure water to reach 20 µL |

20 µL of a formulated reaction solution that contains a template under test is placed in a metal bath, denaturation is performed at 95°C for one minute, and the reaction solution is then immediately placed into a refrigerator at 2°C to 8°C for 2-3 minutes.

The 20 µL of the cooled PCR reaction solution is added into sample pores of a droplet generator cartridge, 70 µL of droplet generator oil is then added into oil pores of the droplet generator cartridge, and finally, the droplet generator cartridge is sealed by using a sealing strip.

The prepared droplet generator cartridge is placed into a droplet generator, so as to start the generation of droplets. After approximate 2 minutes, the preparation of the droplets is completed, the cartridge is taken out, and 40 µL of droplets are carefully transferred from the uppermost row of pores to a 96-pore PCR plate.

### The read of amplification:

After film sealing processing is performed on the 96-pore plate, the 96-pore plate is placed in a thermal cycler for PCR amplification. The used program involves: performing initial denaturation at 95°C for 10 minutes; performing denaturation at 94°C for 30 seconds, and performing annealing at 56°C and elongation for 60 seconds, and implementing 48 cycles in total; performing inactivation at 98°C for 10 minutes; and terminating the reaction at 10°C.

After PCR amplification ends, the 96-pore plate is placed in a droplet analyzer, and an FAM/HEX channel is selected to perform signal reading.

### The analysis of data:

The QuantaSoft analysis software is used to analyze the strength and number of fluorescence signals to obtain the copy number and concentration of the T790M mutant type of exon 20 of EGFR gene, and the results are as shown in Table 14.

**Table 14**

| | Quantitative mean (unit: copies/µL) | |
|---|---|---|
| Length of fragment | High-value sample | Low-value sample |
| 41 bp | 109 | 11.7 |
| 61 bp | 86.2 | 8.4 |
| 97 bp | 50.9 | 5.7 |
| 128 bp | 27.5 | 3.0 |

It can be seen from Table 14 that the shorter the fragment of a target sequence, the higher the capability of sample quantification, that is, the higher the capability of detecting a fragment of a T790M mutant type.

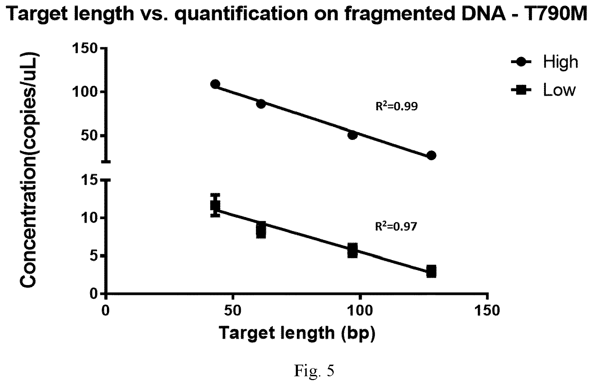

Linear regression analysis is performed on the data in Table 14, and an analysis result is as shown in Fig. 5. It can be seen that, when a mutant-type high-value sample and low-value sample are detected, the detection capability is negatively correlated with the length of the target sequence. The shorter the target sequence, the more the mutant-type fragments are detected, and the higher the detection capability; otherwise, the lower the detection capability.

## Claims

1. A combination for detection nucleic acids, the combination comprising:
an upstream primer, the 3'-end of which is provided with a target sequence binding region, wherein the 3'-end of the target sequence binding region is capable of being complementary to a specific detection site of a target sequence;
a downstream primer, the 3'-end of which is provided with a target sequence binding region, wherein the 3'-end of the target sequence binding region is also capable of being complementary to the same specific detection site; and
a signal oligonucleotide, wherein the signal oligonucleotide is modified with a first detection group and a second detection group, with the first detection group and the second detection group generating a change in signal by means of a change in distance; and
the signal oligonucleotide is designed as a part of the upstream primer and/or the downstream primer, and to be located upstream of the target sequence binding region; or the upstream primer and/or the downstream primer further comprises a signal detection region upstream of the target sequence binding region, and the signal oligonucleotide is designed to be independent of the upstream primer and/or the downstream primer and have the same sequence as the signal detection region; and
wherein the signal detection region is located upstream of the target sequence binding region of the upstream primer and/or the downstream primer, and is designed to be incapable of being complementarily paired with the target sequence.

2. The combination according to claim 1, wherein the upstream primer and/or the downstream primer further comprise a signal detection region upstream of the target sequence binding region, and the signal oligonucleotide is designed to be independent of the upstream primer and/or the downstream primer; and
the signal oligonucleotide is designed to comprise a first stem region, a loop region, a second stem region and an anchor region, sequentially from 5' to 3',
wherein the first stem region is designed to be partially or completely complementary to the second stem region, and the anchor region is located at the 3'-end of the signal oligonucleotide and is designed to be partially or completely identical to the signal detection region; and
wherein the first detection group modifies the first stem region, and the second detection group modifies the second stem region, a position between the second stem region and the anchor region, or a non-3'-end of the anchor region.

3. The combination according to claim 2, wherein the second stem region and the anchor region are at an interval of one to five bases.

4. The combination according to claim 2, wherein the first detection group is located in the first stem region, and the second detection group is located between the second stem region and the anchor region.

5. The combination according to claim 1, wherein the upstream primer and/or the downstream primer further comprise a signal detection region upstream of the target sequence binding region, and the signal oligonucleotide is designed to be independent of the upstream primer and/or the downstream primer,
wherein the signal oligonucleotide is designed as a flexible oligonucleotide, and wherein the first detection group and the second detection group are located at a non-3'-end of the signal oligonucleotide.

6. The combination according to claim 5, wherein the first detection group and the second detection group are 5-25 nt away from each other.

7. The combination according to claim 1, wherein the upstream primer and/or the downstream primer further comprise a signal detection region upstream of the target sequence binding region, and the signal oligonucleotide is designed to be independent of the upstream primer and/or the downstream primer; and
the combination further comprises a second primer, and all or part of the sequence of the second primer are the same as that of the signal detection region;
wherein the region on the signal detection region that is the same as the signal oligonucleotide is located downstream of the region thereon that is the same as the second primer, and
wherein the change in distance is made by means of the hydrolysis of the signal oligonucleotide.

8. The combination according to claim 1, wherein the signal oligonucleotide is designed as a part of the upstream primer and/or the downstream primer, and is located upstream of the target sequence binding region, and
the signal oligonucleotide comprises a first stem region, a loop region and a second stem region, sequentially from 5' to 3',
wherein the first stem region is designed to be partially or completely complementary to the second stem region, and
wherein the first detection group is located in the first stem region, and the second detection group is located in the second stem region.

9. The combination according to any one of claims 1-8, wherein the specific detection site is a mutation site.

10. A method for detection nucleic acids, the method comprising the following steps: mixing the combination of any one of claims 1-9, an amplification reagent and a sample from a subject;
amplifying a target sequence, which may be present in the sample;
obtaining a generated change in signal; and
determining, according to the obtained change in signal, whether target nucleic acids are present in the sample.

11. The method according to claim 10, wherein the target nucleic acids are quantified while whether the target nucleic acids are present in the sample is determined.

12. The method according to claim 10 or 11, wherein the sample is peripheral blood or another type of sample that contains a fragmented nucleic acid target.

13. The method according to claim 10 or 11, wherein the subject has cancer or is a subject who is suspected to have cancer.

14. The method according to claim 10 or 11, wherein the nucleic acids are ctDNA.

15. A kit, comprising the upstream primer, the downstream primer and the signal oligonucleotide defined in any one of claims 1-9.
